# EUROPEAN PATENT SPECIFICATION

(11) **EP 2 575 623 B1**
(45) Date of publication and mention of the grant of the patent: **20.04.2016**
(21) Application number: 10778725.1
(22) Date of filing: 29.09.2010
(51) Int. Cl.: A61B 8/00, A61B 8/08, A61B 18/14, A61B 17/34, A61B 90/00

(54) **ULTRASONIC VISUALIZATION OF PERCUTANEOUS NEEDLES, INTRAVASCULAR CATHETERS AND OTHER INVASIVE DEVICES**
ULTRASCHALLABBILDUNG PERKUTANER NADELN, INTRAVASKULÄRER KATHETER UND ANDERER INVASIVER VORRICHTUNGEN
VISUALISATION ULTRASONORE D'AIGUILLES PERCUTANÉES, DE CATHÉTERS INTRAVASCULAIRES ET AUTRES DISPOSITIFS INVASIFS

(30) Priority: 07.06.2010 US 745374
(43) Date of publication of application: 10.04.2013
(73) Proprietor: Koninklijke Philips N.V., 5656 AE Eindhoven (NL)
(72) Inventor: FERNANDEZ, Anna, Teresa, NL-5656 AE Eindhoven (NL); XIE, Hua, NL-5656 AE Eindhoven (NL); HALL, Christopher, Stephen, NL-5656 AE Eindhoven (NL); GAUTHIER, Thomas, NL-5656 AE Eindhoven (NL); SOKKA, Shunmugavelu, NL-5656 AE Eindhoven (NL)
(74) Representative: Damen, Daniel Martijn
(86) International application number: PCT/IB2010/054383
(87) International publication number: WO 2011/154782

(56) References cited:
- WO-A1-01/97698
- WO-A1-94/03110
- WO-A1-02/096278
- WO-A1-2009/069038
- WO-A2-2004/082749
- US-A1- 2004 002 653
- US-A1- 2006 241 451

## Description

### FIELD OF THE INVENTION

This invention relates to medical diagnostic ultrasonic imaging and, in particular, to ultrasonic imaging of invasive devices inserted into the body during a medical procedure.

### BACKGROUND OF THE INVENTION

Many invasive procedures are augmented by noninvasive imaging, particularly when an invasive device is inserted into the body to treat a target tissue. For instance, a biopsy needle is often visually assisted by ultrasound so that a target tissue or cell mass is accessed directly and positively by the needle. The clinician can visually observe the path of the needle as it is inserted into the body to sample or remove suspect pathology inside the body. Another example is an r.f. ablation needle, which is inserted into the body to engage a tumor which is to be grasped or surrounded by the tines of the needle before r.f. energy is applied. The visualization assures that the needle tines have correctly and fully engaged the tumor. A further example is an intravascular catheter, which may be guided over long distances inside the body from its access point at a femoral artery, for instance. The tip of the catheter may be observed by ultrasonic imaging to assure its accurate placement in a targeted chamber of the heart, for example.

From WO 2009/069038 A1 an invasive medical device is known including a fluid path microbubbles which is imaged by ultrasound during use of the device. The fluid path extends through the device, preferably to the distal end of the device, so that the diffuse reflection of the ultrasound from the microbubbles can be received to image the location of the device. The fluid path can be open, terminating at the tip device, or can be a close path of a circulating microbubble fluid used for imaging and/or cooling.

### SUMMARY OF THE INVENTION

However, it can often be difficult to clearly visualize an invasive device in an ultrasound field. Invasive devices like needles are generally inserted into the body in close proximity to the ultrasound probe. These solid instruments are specular reflectors which present a shallow angle of incidence to the ultrasound beams from the probe. Many times the position of the instrument is virtually parallel to the beam directions. Consequently the sound waves can be reflected deeper into the body rather than providing a strong return signal. As a result the device will present a broken or indistinct appearance in the ultrasound image. Attempts have been made to mitigate this problem such as forming a diffraction grating near the tip of a needle as described in US Pat. 4,401,124 (Guess et al.), but this approach is also angle-dependent. Another approach is to Doppler demodulate the motion of the needle as described in US Pat. 5,095,910 (Powers), but this technique is only effective while the needle is moving. Another Doppler approach is to inject a steady flow of fluid into the body and detect the locus of the injecting device from Doppler sensing of the flow rate of the fluid flow, as described in international publication no. WO 2004/082749 (Keenan et al.) Accordingly it is desirable to be able to clearly image an invasive instrument with ultrasound regardless of its position in the sound field and without the need to create motional effects.

The invention is defined in independent claim 1. In accordance with the principles of the present invention, an invasive medical instrument which is to be imaged by ultrasound utilizes a fluid of microbubbles for improved visualization. Microbubbles are encapsulated gaseous particles or gaseous pre-cursors suspended in fluid. The microbubbles can be very small, on the order of tens of microns, and carried in saline or other fluids. The fluid can be continuously flowing or circulated through the instrument in a closed path, or can exit the distal end of the instrument to enable the tip of the device to be clearly located in the image. The microbubbles in the fluid present diffuse reflectors of harmonic signals to the impinging ultrasound waves, enabling the device to be clearly imaged regardless of its position in the ultrasound field. The harmonic signal returns clearly segment the locus of the microbubbles around the distal tip of the instrument from the fundamental signals returned from other scatterers, and are produced without the need for motional effects.

### BRIEF DESCRIPTION OF THE DRAWINGS

In the drawings:
FIGURE 1 is a cross-sectional view of an invasive medical device with an open microbubble fluid path constructed in accordance with the principles of the present invention.
FIGURE 1a is an enlarged view of the tip of the needle of FIGURE 1 showing the needle tip surrounded by microbubbles.
FIGURE 2 is a cross-sectional view of an invasive medical instrument with a closed loop microbubble fluid path circulating fluid to and from the tip of the instrument.
FIGURE 2a is a cross-sectional view of the needle sheath of FIGURE 2 showing the path connecting the supply and return fluid paths.
FIGURE 3 is a cross-sectional view of an r.f. ablation needle with the needle tines ultrasonically illuminated with a flow of microbubbles.
FIGURE 4 is a block diagram of an ultrasonic imaging system adapted to image microbubbles associated with an invasive medical device.
FIGURE 5 is a flow chart illustrating exemplary steps in performing r.f. ablation with the needle of FIGURE 3 in accordance with the principles of the present invention.
FIGURE 6 illustrates an ultrasonic imaging system in block diagram form which is adapted to image harmonic signal returned from microbubbles in accordance with the present invention.
FIGURE 7 illustrates a preferred ultrasonic imaging system in block diagram form which is adapted to image harmonic signal returned from microbubbles in accordance with the present invention.

### DETAILED DESCRIPTION OF THE EMBODIMENTS

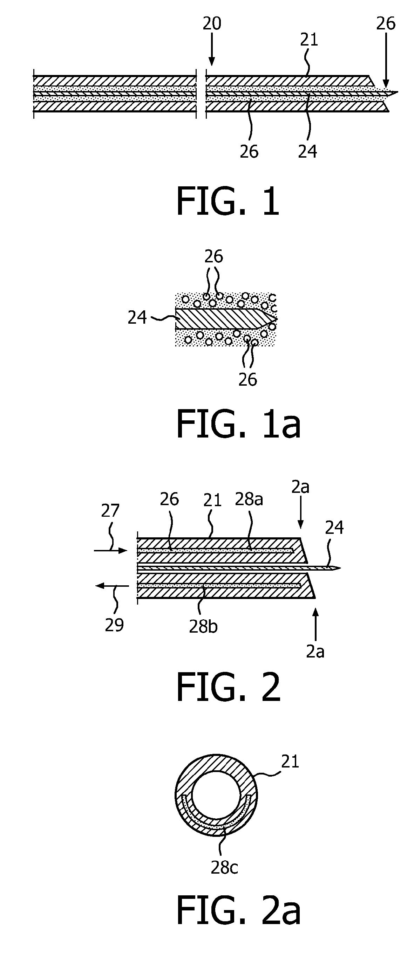

Referring first to FIGURE 1, an invasive medical instrument, here shown as a biopsy needle 20, is constructed in accordance with the principles of the present invention. The needle 20 comprises an outer sheath 21, sometimes referred to as the insertion needle, which is inserted into the body toward tissue which is to be biopsied or otherwise probed by the instrument. The outer sheath 21 carries a stylet or needle or other tool 24. When the outer sheath 21 is inserted into the body in proximity to the tissue to be probed, the stylet 24 is extended to pierce the suspect tissue and acquire a sample or perform some other operation on the tissue. In some procedures the insertion needle is removed from the body while the stylet or tool 24 is left in place for subsequent manipulation.

In accordance with the principles of the present invention a flow 26 of a fluid containing microbubbles is supplied through the lumen of the needle. In this embodiment the fluid path is open at the distal tip of the insertion needle and the microbubble fluid can flow out of the tip of the insertion needle 21 and surround the tip of the stylet 24. The microbubble fluid may be any biocompatible fluid such as water or saline solution which contains gaseous particles. The gaseous particles may be air bubbles, encapsulated microbubbles, phase-converted nanoparticles, agitated saline, or ultrasonic contrast agent to name a few candidates. The microbubbles are high echogenic particles which provide relatively strong echo returns from impinging ultrasound waves. In comparison with a needle which is a specular reflector from which the strength of the returning echoes is highly angle-dependent, the spherical microbubbles or other particles will return a significant echo signal with little or no angle dependency. Thus the bath 26 of microbubbles which surrounds the tip of the needle 24 will illuminate the tip location and the shaft of the needle and stylet regardless of the angle of the needle. The needle, on the other hand, may cause impinging ultrasound to glance off at the angle of the needle and scatter deeper into the tissue rather than return to the ultrasound transducer, resulting in dropout and an irregular appearance of the needle and stylet in the ultrasound image. This difficulty is resolved by the microbubble fluid path which returns ultrasound from along the length of the needle with little or no angle dependency or image dropout.

FIGURE 1a is an enlarged view of the tip of the stylet 24, which illustrates the microbubbles 26 surrounding the tip of the instrument. The echo returns from the microbubbles 26 will thus illuminate the location of the tip in the ultrasound image.

FIGURE 2 illustrates another embodiment of the present invention in cross-section. The medical instrument illustrated in this embodiment has a closed fluid path for the microbubble solution. Such an embodiment is suitable for a catheter or other device which is inserted into the vasculature of the body, and also for instruments which utilize a cooling fluid for the tip of the instrument, in which case the cooling fluid will contain the microbubbles. An r.f. ablation catheter used to ablate the endocardial wall of the heart in cardiac resynchronization therapy may also have a fluid path suitable for carrying a microbubble solution in accordance with the present invention. In the example of FIGURE 2 the outer sheath 21 contains the microbubble fluid 26 in a supply fluid path 28a. The microbubble fluid 26 in this path 28a travels to the tip of the instrument from a source of supply as indicated by arrow 27. On the other side of the sheath 21 is a return fluid path 28b, through which the microbubble fluid returns to a point outside the instrument as indicated by the arrow 29. Near the tip of the sheath is a connecting path 28c through which fluid flows from the supply path 28a to the return path 28b, as shown in FIGURE 2a. An advantage of a closed fluid path instrument is that the microbubble fluid does not have to meet the stringent requirements of a fluid which is injected into the body from an open fluid path instrument.

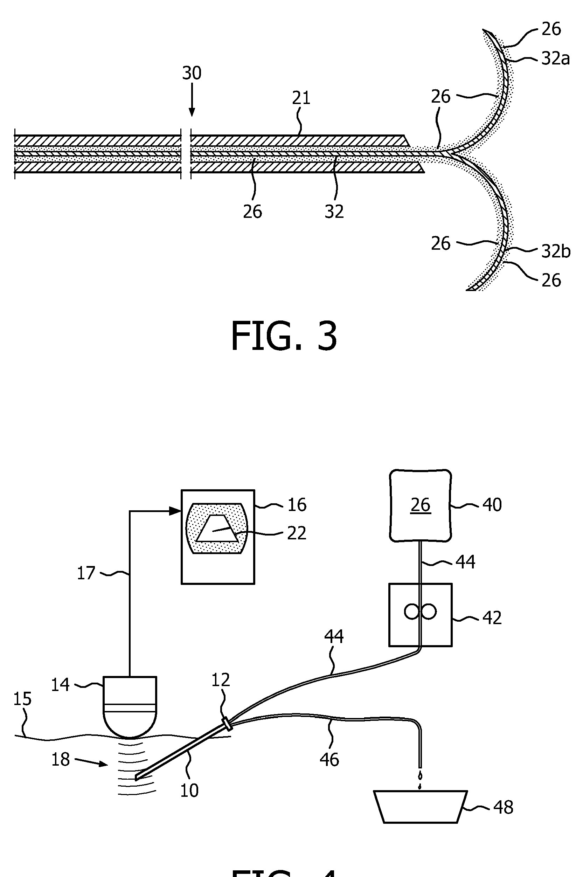

FIGURE 3 illustrates an example of an r.f. ablation needle 30 constructed in accordance with the principles of the present invention for treating tumors with radio frequency energy. In this example the needle sheath 21 carries an r.f. ablation needle with many small, curved tines 32a,32b at the distal tip. The needle sheath 21 is inserted into the body until the distal end of the sheath approaches a tumor which is to be treated. The needle is then deployed by extending the needle from the end of the sheath as shown in FIGURE 3. As the needle is deployed the many curved tines 32a,32b, etc. are disposed uniformly through the volume of the tumor. However, variations in the density or stiffness of the tumor tissue can cause the small tines to deflect from their intended paths and be non-uniformly distributed in the tumor. The clinician will check for this problem by imaging the deployed tines with ultrasound. However, as is apparent, the curved tines 32a,32b will scatter ultrasound at many angles, which can cause dropout and an indistinct view of the fine needle tines in the ultrasound image. In accordance with the principles of the present invention, a microbubble fluid 26 surrounds the needle inside the shaft 21 and will travel through the apertures in the tumor pierced by the tines as shown in FIGURE 3. The echo returns from the microbubbles adjacent the needle tines 32a,32b will not be angle dependent and will enable the fine tines of the r.f. ablation needle to be clearly visualized in the ultrasound image.

FIGURE 4 illustrates an invasive medical device 10 and an ultrasound system 14,16 constructed in accordance with the principles of the present invention. In this example a needle 10 is inserted through the surface 15 of the body toward a target pathology. As the needle 10 is inserted its progress is monitored by an ultrasound probe 14 which transmits ultrasound waves 18 to the needle and receives returning echoes for image formation. The transduced echo signals are coupled by a cable 17 to the mainframe 16 of the ultrasound system for processing and display. The echo signals are processed to produce an ultrasound image 22 which shows the location of the needle in the body.

In accordance with the principles of the present invention, a bag 40 contains a microbubble fluid 26. The microbubble fluid is supplied to a fluid coupling 12 of the needle 10 by a tube 44. A pump 42 such as an infusion pump or roller pump will gently pump the microbubble fluid from the supply bag 40 to the needle. The pump pressure need be only sufficient to cause the microbubble fluid to reach the tip of the needle, and to enable passage alongside a deployed tool through the aperture cut by the tool, such as the tines of an r.f. ablation needle. Thus, the fluid pressure need only be sufficient to overcome the occluding pressure of the tissue which surrounds the tines, for example. In this example a return tube 46 is coupled to the fluid coupling 12 through which returning fluid is expelled into a container 48 for disposal. A return tube will be desirable for a closed path system when the microbubble fluid is continuous supplied to the tip of the instrument as for cooling, for example. A return tube may also be desirable for an open path system in which a supply of fresh microbubble fluid is continuously supplied to the instrument.

In other embodiments the microbubble fluid bag 26 and the pump 42 may comprise a syringe pump with the microbubble fluid contained within a syringe which is operated by the syringe pump. The microbubble fluid can be supplied by the pump system which is a part of an r.f. ablation device or by any other pumping or irrigation subsystem that is part of the invasive device. The flow of microbubble fluid may be controlled by the ultrasonic imaging system, which controls the delivery of fluid for improved imaging, either with or without operator involvement. For example, automatic, semi-automatic or manual image analysis may detect a poor image of the invasive device and call for a greater or predetermined (e.g., a pulsatile flow) delivery of microbubble fluid.

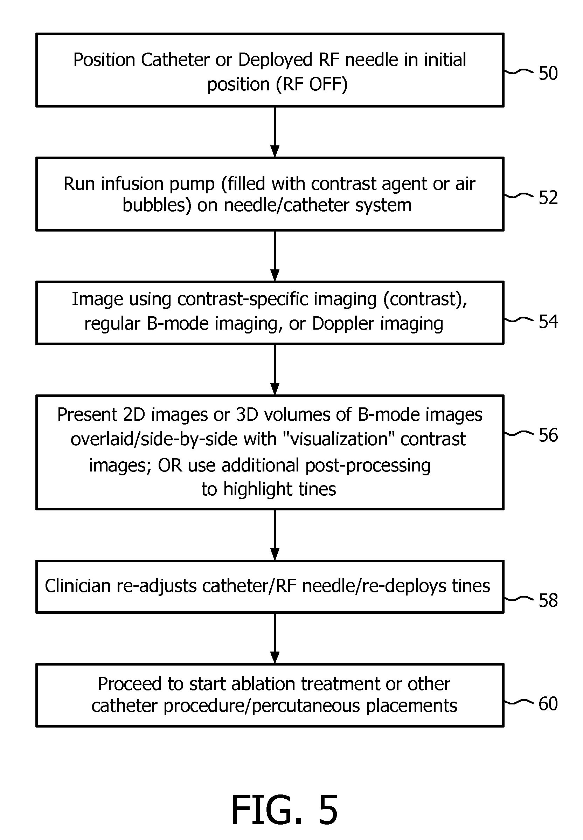

FIGURE 5 is an example of a procedure for using an r.f. needle in accordance with the present invention. In step 50 a catheter or r.f. needle is inserted into an initial position adjacent to target tissue. In the case of an r.f. ablation procedure the needle tines are deployed into the tumor. An infusion pump is then operated in step 52 to fill the catheter or needle, and/or the space in the tissue adjacent the deployed instrument, with the microbubble fluid. Ultrasonic imaging is then performed in step 54 in an imaging mode which illuminates the microbubbles in the image such as contrast-specific imaging, B-mode imaging, or Doppler imaging. In step 56 the ultrasound images are presented to the clinician performing the procedure. The images can be 2D images or 3D images (desirable for seeing the deployed tines of an r.f. ablation needle) and the microbubble visualization images can be overlaid on a structural B-mode image or shown side-by-side. Additional post-processing may be performed as desired to highlight needle tines such as speckle-reduction processing. After viewing the location of the needle, catheter, or needle tines with the microbubble fluid, the clinician may adjust the position of the invasive instrument as indicated in step 58. Once the instrument has been adjusted to its most beneficial and effective position in the body, the intended treatment is performed in step 60.

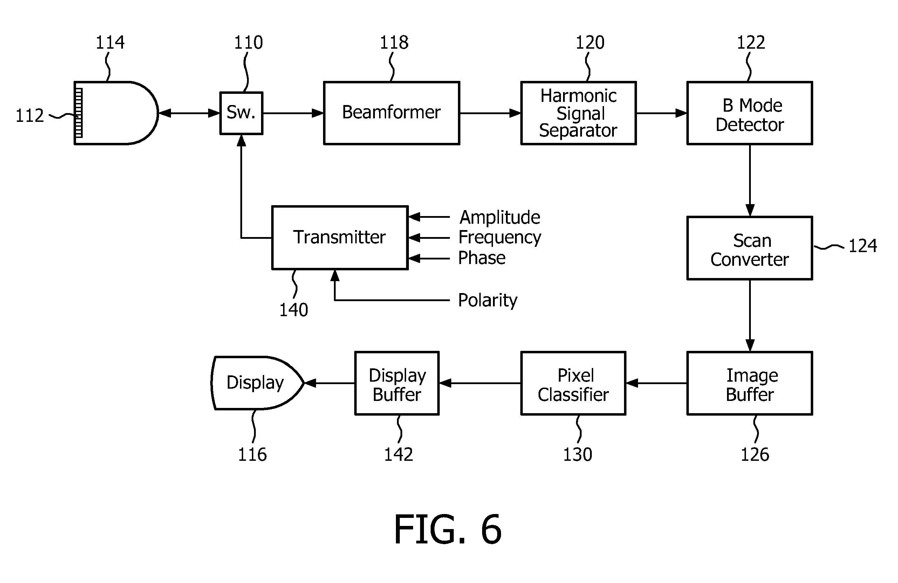

FIGURE 6 illustrates in block diagram form an ultrasonic diagnostic imaging system constructed in accordance with the principles of the present invention. The system operates by scanning a two or three dimensional region of the body being imaged with ultrasonic transmit beams. As each beam is transmitted along its steered path through the body, the tissue and microbubbles in the body return echo signals with linear and nonlinear (or fundamental and harmonic) components corresponding to the transmitted frequency components. The transmit signals are reflected from the microbubbles of a contrast agent which exhibit a nonlinear response to ultrasound. The nonlinear response will cause the echo signals returned from the contrast agent to contain nonlinear components.

The ultrasound system of FIGURE 6 utilizes a transmitter 140 which transmits waves or pulses of a selected modulation characteristic in a desired beam direction for the return of harmonic echo components from scatterers within the body. The transmitter is responsive to a number of control parameters which determine the characteristics of the transmit beams as shown in the drawing, including the frequency components of the transmit beam, their relative intensities or amplitudes, and the phase or polarity of the transmit signals. The transmitter is coupled by a transmit/receive switch 110 to the elements of an array transducer 112 of a probe 114. The array transducer can be a one dimensional array for planar (two dimensional) imaging or a two dimensional array for two dimensional or volumetric (three dimensional) imaging.

The transducer array 112 receives echoes from the body containing linear and nonlinear components which are within the transducer passband. These echo signals are coupled by the switch 110 to a beamformer 118 which appropriately delays echo signals from the different transducer elements, then combines them to form a sequence of coherent echo signals along the beam from shallow to deeper depths. Preferably the beamformer is a digital beamformer operating on digitized echo signals to produce a sequence of discrete coherent digital echo signals from a near field to a far field depth of field. The beamformer may be a multiline beamformer which produces two or more sequences of echo signals along multiple spatially distinct receive scanlines in response to the transmission of one or more spatially distinct transmit beams, which is particularly useful for 3D imaging. The beamformed echo signals are coupled to a harmonic signal separator 120.

The harmonic signal separator 120 can separate the linear and nonlinear components of the echoes signal in various ways. One way is by filtering. Since certain nonlinear components such as the second harmonic are at a different frequency band (2fₒ) than the fundamental transmit frequencies (fₒ), the harmonic signals which are the signature of microbubbles can be separated from the linear components by band pass or high pass filtering. There are also a number of multiple pulse techniques for separating nonlinear components which are generally referred to as pulse inversion techniques. In pulse inversion the image field is insonified by the transmission of multiple, differently modulated transmit signals in each beam direction, returning multiple echoes from the same location in the image field. The transmit signals may be modulated in amplitude (as described in U.S. Pat. 5,577,505 (Brock Fisher et al.)), phase or polarity (as described in U.S. Pat. 5,706,819 (Hwang et al.)), or a combination thereof. When the received echoes from a common location are combined, the linear signal components are canceled and the nonlinear signal components reinforce each other (or *vice versa,* as desired), thereby producing separated nonlinear (e.g., harmonic) echo signals for imaging.

The echo signals are detected by a B mode detector 122. An advantage of the inventive technique over the prior art techniques discussed above is that Doppler processing is not necessary. The present invention may be carried out using Doppler processing if desired in a given embodiment, however the use of B mode signals avoids the reduction in real time frame rate caused by the acquisition of long Doppler ensembles. The detected echo signals are then converted into the desired image format such as a sector or pyramidal image by a scan converter 124. The scan converted image is temporarily stored in an image buffer 126 from which it can undergo further processing. The image data is coupled to a pixel classifier where the strong harmonic signal returns from microbubbles can be segmented and, if desired, highlighted in the image as by coloring or brightness control, *e.g.,* to emphasize the small pool of microbubbles around the tip of the needle. The image of the needle with its tip clearly indicated by the harmonic signals from surrounding microbubbles is coupled to a display buffer 142, from which it is shown on a display 116.

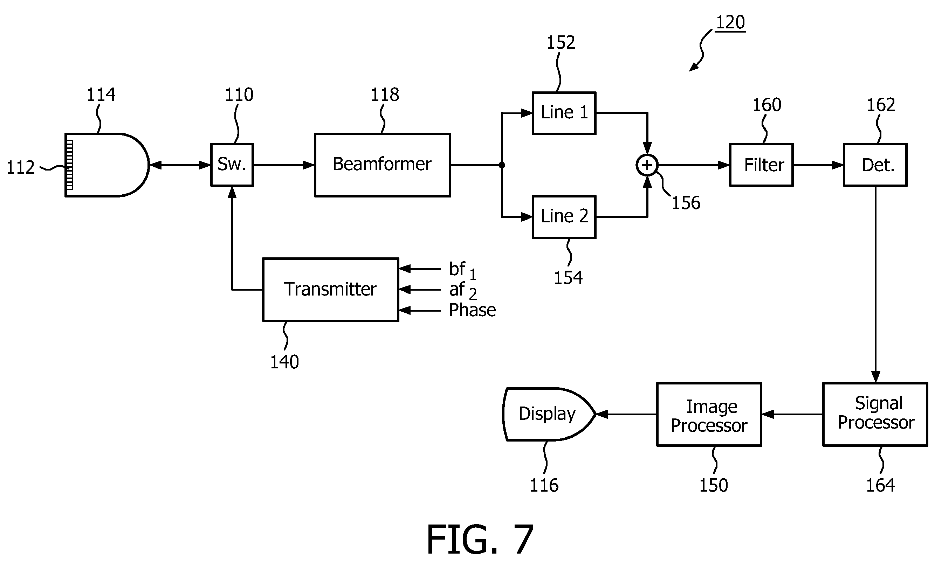

FIGURE 7 illustrates another ultrasonic diagnostic imaging system in block diagram form which performs harmonic signal separation by the techniques of two-pulse phase or polarity pulse inversion or difference frequency detection. In FIGURE 7 the transducer array 112 receives echoes of nonlinear signal from microbubbles which may comprise harmonic or difference frequency components. These echo signals are coupled by the switch 110 to the beamformer 118 which appropriately delays echo signals from the different elements then combines them to form a sequence of echo signals along the beam from shallow to deeper depths. The beamformer may be a multiline beamformer which produces two or more sequences of echo signals along multiple spatially distinct receive scanlines in response to a single transmit beam. The beamformed echo signals are coupled to a nonlinear signal separator 120. In this embodiment the separator 120 is a pulse inversion processor which separates the nonlinear signals including second harmonic and difference frequency components by the pulse inversion technique. Since the harmonic and difference frequency signals are developed by nonlinear effects, they may advantageously be separated by pulse inversion processing. For pulse inversion the transmitter has another variable transmit parameter which is the phase, polarity or amplitude of the transmit pulse as shown in the drawing. The ultrasound system transmits two or more beams of different transmit polarities controlled by the transmitter 140 which exhibit different amplitudes and/or phases. Another alternative is to transmit the beams with two different major component frequencies, shown as bf₁ and af₂, which are intermodulated by their passage through tissue to produce a difference frequency (f₁-f₂). For a two pulse embodiment, the scanline echoes received in response to the first transmit pulse are stored in a Line1 buffer 152. The scanline echoes received in response to the second transmit pulse are stored in a Line2 buffer 154 and then combined with spatially corresponding echoes in the Line1 buffer by a summer 156. Alternatively, the second scanline of echoes may be directly combined with the stored echoes of the first scanline without buffering. As a result of the different phases or polarities of the transmit pulses, the out of phase fundamental (linear) echo components will cancel and the nonlinear second harmonic or difference frequency components, being in phase, will combine to reinforce each other, producing enhanced and clearly segmented nonlinear harmonic difference frequency signals. The nonlinear harmonic or difference frequency signals may be further filtered by a filter 160 to remove undesired signals such as those resulting from operations such as decimation. The signals are then detected by a detector 162, which may be an amplitude or phase detector. The echo signals are then processed by a signal processor 164 for subsequent grayscale, Doppler or other ultrasound display, then further processed by an image processor 150 for the formation of a two dimensional or three dimensional image of the needle and the nonlinear (harmonic or difference frequency) signals returned from the microbubbles. The resultant display signals are displayed on the display 116.

## Claims

1. An ultrasonic diagnostic imaging system for imaging an invasive medical device comprising:
an invasive medical device having a fluid path, wherein the fluid path extends to a distal tip of the medical device;
a source of microbubble fluid (26) coupled to the fluid path and providing the microbubble fluid (26) for the fluid path;
an ultrasound probe (14, 114) for scanning an ultrasonic image field which includes the location of the invasive medical device; and
an ultrasound imaging system, coupled to the ultrasound probe (14, 114) **characterized in that** the ultrasound imaging system is responsive to nonlinear ultrasound signals received by the probe from the microbubbles of the fluid around the distal tip, wherein the ultrasound imaging system comprises a signal separator (120) for separating linear and nonlinear ultrasound signal components from the echo signals received from the ultrasound probe and a display (116) for displaying an image of the microbubble fluid and the distal tip of the invasive medical device on the basis of the separated nonlinear signal components.

2. The ultrasonic diagnostic imaging system of Claim 1, wherein the medical device further includes an insertion portion and a tool which is extendable from the distal end of the insertion portion, wherein the fluid path extends to the distal end of the insertion portion and is open.

3. The ultrasonic diagnostic imaging system of Claim 1, wherein the medical device further includes an insertion portion having a distal end, wherein the fluid path further includes a supply path extending to the distal end and a return path extending from the distal end.

4. The ultrasonic diagnostic imaging system of Claim 1, wherein the invasive medical device further comprises an r.f. ablation device for one of applying r.f. energy to a tumor or r.f. energy to a chamber of the heart.

5. The ultrasonic diagnostic imaging system of Claim 1,
the invasive medical device further having a coupling (12) to the fluid path; the system further comprising:
a fluid pump (42) coupled between the source of microbubble fluid and the medical device coupling (12) which act to supply the microbubble fluid (26) to the fluid path of the device; and
a return fluid path (46) coupled to the medical device coupling (12) for removal of microbubble fluid (26) from the medical device.

6. The ultrasonic diagnostic imaging system of Claim 1, wherein the signal separator comprises a frequency filter for separating the fundamental frequency and the harmonic frequency of the echo signals as the linear and nonlinear ultrasound signal components.

7. The ultrasonic diagnostic imaging system of Claim 1, wherein the ultrasonic imaging system is capable of operating in one of the contrast-specific imaging, B-mode imaging, or Doppler imaging modes.

8. The ultrasonic diagnostic imaging system of Claim 6, wherein the fluid pump (42) comprises an infusion pump.

9. The ultrasonic diagnostic imaging system of Claim 1, wherein a distal end of the medical device is insertable into tissue, and wherein the fluid path is open to allow microbubble fluid to flow to the tissue.

10. The ultrasonic diagnostic imaging system of Claim 1, wherein the fluid path of the medical device extends to a distal end of the medical device, wherein the fluid path is a closed fluid path within the portion of the medical device that is insertable into tissue.

11. The ultrasonic diagnostic imaging system of Claim 1, wherein the source of microbubble fluid comprises a bag of microbubbles in saline solution.

12. The ultrasonic diagnostic imaging system of Claim 1, wherein the microbubble fluid comprises one of air bubbles, encapsulated microbubbles, microbubbles of phase-converted nanoparticles, microbubbles of agitated saline, or microbubbles of ultrasonic contrast agent.

13. The ultrasonic diagnostic imaging system of Claim 6, wherein the ultrasonic imaging system is operable to control the delivery of microbubble fluid by the fluid pump (42).

## Patentansprüche

1. System zur diagnostischen Ultraschallbildgebung zum Darstellen einer invasiven medizinischen Vorrichtung, wobei das System Folgendes umfasst:
eine invasive medizinische Vorrichtung mit einem Flüssigkeitspfad, wobei der Flüssigkeitspfad zu einer distalen Spitze der medizinischen Vorrichtung verläuft;
eine Quelle einer Mikrobläschenflüssigkeit (26), die mit dem Flüssigkeitspfad gekoppelt ist und die Mikrobläschenflüssigkeit (26) für den Flüssigkeitspfad bereitstellt;
eine Ultraschallsonde (14, 114) zum Scannen eines Ultraschallbildfelds, das den Ort der invasiven medizinischen Vorrichtung umfasst; und
ein Ultraschallbildgebungssystem, das mit der Ultraschallsonde (14, 114) gekoppelt ist und **dadurch gekennzeichnet ist, dass** das Ultraschallbildgebungssystem auf nichtlineare Ultraschallsignale reagiert, die durch die Sonde von den Mikrobläschen der Flüssigkeit um die distale Spitze herum empfangen wurden, wobei das Ultraschallbildgebungssystem einen Signalseparator (120) zum Trennen der linearen und nichtlinearen Signalkomponenten der von der Ultraschallsonde empfangenen Echosignalen und eine Anzeige (116) zum Anzeigen eines Bilds der Mikrobläschenflüssigkeit und der distalen Spitze der invasiven medizinischen Vorrichtung auf der Basis der getrennten nichtlinearen Signalkomponenten umfasst.

2. System zur diagnostischen Ultraschallbildgebung nach Anspruch 1, wobei die medizinische Vorrichtung einen Einführungsteils und ein Instrument umfasst, das von dem distalen Ende des Einführungsteils aus ausziehbar ist, wobei der Flüssigkeitspfad zu dem distalen Ende des Einführungsteils verläuft und offen ist.

3. System zur diagnostischen Ultraschallbildgebung nach Anspruch 1, wobei die medizinische Vorrichtung weiterhin einen Einführungsteil mit einem distalen Ende umfasst, wobei der Flüssigkeitspfad weiterhin einen Zuführungspfad, der zum distalen Ende verläuft, und einen Rücklaufpfad, der von dem distalen Ende ausgeht, umfasst.

4. System zur diagnostischen Ultraschallbildgebung nach Anspruch 1, wobei die invasive medizinische Vorrichtung weiterhin eine HF-Ablationsvorrichtung zum Anwenden von HF-Energie auf einen Tumor oder von HF-Energie auf eine Herzkammer umfasst.

5. System zur diagnostischen Ultraschallbildgebung nach Anspruch 1,
wobei die invasive medizinische Vorrichtung weiterhin eine Kopplung (12) zum Flüssigkeitspfad hat;
wobei das System weiterhin Folgendes umfasst:
eine Flüssigkeitspumpe (42), die zwischen die Quelle von Mikrobläschenflüssigkeit und die Kopplung (12) der medizinischen Vorrichtung geschaltet ist und dazu dienst, die Mikrobläschenflüssigkeit (26) dem Flüssigkeitspfad der Vorrichtung zuzuführen; und
einen Flüssigkeits-Rücklaufpfad (46), der mit der Kopplung (12) der medizinischen Vorrichtung gekoppelt ist, um Mikrobläschenflüssigkeit (26) aus der medizinischen Vorrichtung zu entfernen.

6. System zur diagnostischen Ultraschallbildgebung nach Anspruch 1, wobei der Signalseparator einen Frequenzfilter umfasst, um die Fundamentalfrequenz und die harmonische Frequenz der Echosignale als die linearen und nichtlinearen Signalkomponenten zu trennen.

7. System zur diagnostischen Ultraschallbildgebung nach Anspruch 1, wobei das Ultraschallbildgebungssystem in der Lage ist, in einem kontrastspezifischen Bildgebungsmodus, einem B-Mode-Bildgebungsmodus oder einem Doppler-Bildgebungsmodus zu arbeiten.

8. System zur diagnostischen Ultraschallbildgebung nach Anspruch 6, wobei die Flüssigkeitspumpe (42) eine Infusionspumpe umfasst.

9. System zur diagnostischen Ultraschallbildgebung nach Anspruch 1, wobei ein distales Ende der medizinischen Vorrichtung in Gewebe einführbar ist und wobei der Flüssigkeitspfad offen ist, damit Mikrobläschenflüssigkeit in das Gewebe fließen kann.

10. System zur diagnostischen Ultraschallbildgebung nach Anspruch 1, wobei der Flüssigkeitspfad der medizinischen Vorrichtung zu einem distalen Ende der medizinischen Vorrichtung verläuft, wobei der Flüssigkeitspfad ein geschlossener Flüssigkeitspfad in dem Teil der medizinischen Vorrichtung ist, der in Gewebe einführbar ist.

11. System zur diagnostischen Ultraschallbildgebung nach Anspruch 1, wobei die Quelle von Mikrobläschenflüssigkeit einen Beutel mit Mikrobläschen in Kochsalzlösung umfasst.

12. System zur diagnostischen Ultraschallbildgebung nach Anspruch 1, wobei die Mikrobläschenflüssigkeit eines von Folgendem umfasst: Luftbläschen, eingekapselte Mikrobläschen, Mikrobläschen von phasenumgewandelten Nanopartikeln, Mikrobläschen von gerührter Kochsalzlösung oder Mikrobläschen von Ultraschallkontrastmittel.

13. System zur diagnostischen Ultraschallbildgebung nach Anspruch 6, wobei das Ultraschallbildgebungssystem betriebsfähig ist, um die Förderung von Mikrobläschenflüssigkeit durch die Flüssigkeitspumpe (42) zu steuern.

## Revendications

1. Système d'imagerie diagnostique ultrasonique pour imager un dispositif médical invasif, comprenant :
un dispositif médical invasif comportant un trajet à fluide, dans lequel le trajet à fluide s'étend jusqu'à un embout distal du dispositif médical ;
une source de fluide à microbulles (26) accouplée au trajet à fluide et fournissant le fluide à microbulles (26) pour le trajet à fluide ;
une sonde à ultrasons (14, 114) pour balayer un champ d'image ultrasonique qui inclut la localisation du dispositif médical invasif ; et
un système d'imagerie à ultrasons, couplé à la sonde à ultrasons (14, 114)
**caractérisé en ce que** le système d'imagerie à ultrasons est sensible à des signaux à ultrasons linéaires reçus par la sonde à partir des microbulles du fluide autour de l'embout distal, dans lequel le système d'imagerie à ultrasons comprend un séparateur de signal (120) pour séparer des composants de signal à ultrasons linéaires et non linéaires à partir des signaux à écho reçus à partir de la sonde à ultrasons et un affichage (116) pour afficher une image du fluide à microbulles et de l'embout distal du dispositif médical invasif en fonction des composants de signal à ultrasons non linéaires séparés.

2. Système d'imagerie diagnostique ultrasonique selon la revendication 1, dans lequel le dispositif médical inclut en outre une portion d'insertion et un outil qui est extensible à partir de l'extrémité distale de la portion d'insertion, dans lequel le trajet à fluide s'étend jusqu'à l'extrémité distale de la portion d'insertion et est ouvert.

3. Système d'imagerie diagnostique ultrasonique selon la revendication 1, dans lequel le dispositif médical inclut en outre une portion d'insertion comportant une extrémité distale, dans lequel le trajet à fluide inclut en outre un trajet d'alimentation s'étendant jusqu'à l'extrémité distale et un trajet de retour s'étendant à partir de l'extrémité distale.

4. Système d'imagerie diagnostique ultrasonique selon la revendication 1, dans lequel le dispositif médical invasif comprend en outre un dispositif d'ablation à RF pour une parmi l'application d'énergie à RF sur une tumeur ou d'énergie à RF sur une chambre du coeur.

5. Système d'imagerie diagnostique ultrasonique selon la revendication 1,
le dispositif médical invasif comportant en outre un accouplement (12) au trajet à fluide ; le système comprenant en outre :
une pompe à fluide (42) accouplée entre la source de fluide à microbulles et l'accouplement de dispositif médical (12) qui sert à fournir le fluide à microbulles (26) au trajet à fluide du dispositif ; et
un trajet à fluide de retour (46) accouplé à l'accouplement de dispositif médical (12) pour l'élimination du fluide à microbulles (26) à partir du dispositif médical.

6. Système d'imagerie diagnostique ultrasonique selon la revendication 1, dans lequel le séparateur de signal comprend un filtre de fréquence pour séparer la fréquence fondamentale et la fréquence harmonique des signaux à écho en tant que composants de signal à ultrasons linéaires et non linéaires.

7. Système d'imagerie diagnostique ultrasonique selon la revendication 1, dans lequel le système d'imagerie à ultrasons est capable de fonctionner dans un parmi les modes d'imagerie spécifique de contraste, d'imagerie de mode B, ou d'imagerie Doppler.

8. Système d'imagerie diagnostique ultrasonique selon la revendication 6, dans lequel la pompe à fluide (42) comprend une pompe à perfusion.

9. Système d'imagerie diagnostique ultrasonique selon la revendication 1, dans lequel une extrémité distale du dispositif médical est insérable dans un tissu, et dans lequel le trajet à fluide est ouvert pour permettre au fluide à microbulles de s'écouler ver le tissu.

10. Système d'imagerie diagnostique ultrasonique selon la revendication 1, dans lequel le trajet à fluide du dispositif médical s'étend jusqu'à une extrémité distale du dispositif médical, dans lequel le trajet à fluide est un trajet à fluide fermé à l'intérieur de la portion du dispositif médical qui est insérable dans le tissu.

11. Système d'imagerie diagnostique ultrasonique selon la revendication 1, dans lequel la source de fluide à microbulles comprend une poche de microbulles dans une solution saline.

12. Système d'imagerie diagnostique ultrasonique selon la revendication 1, dans lequel le fluide à microbulles comprend des bulles d'air, ou des microbulles encapsulées, ou des microbulles de nanoparticules converties en phase, ou des microbulles de solution saline agitée, ou des microbulles d'agent de contraste ultrasonique.

13. Système d'imagerie diagnostique ultrasonique selon la revendication 6, dans lequel le système d'imagerie à ultrasons est utilisable pour commander la distribution de fluide à microbulles par la pompe à fluide (42).
